(19) Europäisches Patentamt
European Patent Office
Office européen des brevets

(11) Publication number: **0 516 181 A1**

# (12) EUROPEAN PATENT APPLICATION

(21) Application number: **92112113.3**

(22) Date of filing: **12.10.88**

(51) Int. Cl.5: **C07C 405/00**, C07C 311/20, C07C 311/10, C07C 311/12, C07D 303/02, C07D 493/08, C07D 331/02, A61K 31/557, //(C07D493/08,307:00,307:00)

This application was filed on 15 - 07 - 1992 as a divisional application to the application mentioned under INID code 60.

(30) Priority: **23.10.87 JP 266501/87**
**09.08.88 JP 197180/88**

(43) Date of publication of application:
**02.12.92 Bulletin 92/49**

(60) Publication number of the earlier application in accordance with Art.76 EPC: **0 312 906**

(84) Designated Contracting States:
**AT BE CH DE ES FR GB GR IT LI LU NL SE**

(71) Applicant: **ONO PHARMACEUTICAL CO., LTD.**
**No. 14, Doshomachi 2-chome Higashi-ku**
**Osaka-shi Osaka(JP)**

(72) Inventor: **Arai, Yoshinobu**
**5-8-505, Wakayamadai 1, Shimamoto-cho**
**Mishima-gun, Osaka(JP)**
Inventor: **Hamanaka, Nobuyuki**
**11-38, Hiroshiki, Ohyamazaki-cho**
**Otokuni-gun, Kyoto(JP)**
Inventor: **Miyazaki, Tohru**
**Dainiminasehaitsu 501, 2-6, Minase 2**
**Shimamoto-cho**
**Mishima-gun, Osaka(JP)**

(74) Representative: **Henkel, Feiler, Hänzel & Partner**
**Möhlstrasse 37**
**W-8000 München 80(DE)**

## (54) Novel sulfonamide derivatives.

(57) A sulfonamide derivative of the general formula:

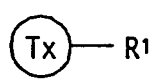

(I)

wherein, $R^1$ represents

(i) $COOR^{11}$,
(ii) $CH_2OR^{12}$ or
(iii) $CONR^{13}R^{14}$,

wherein

$R^{11}$ represents a carbocyclic ring unsubstituted or substituted by an alkyl or alkoxy group of from 1 to 4 carbon atom(s) or halogen atom, or steroid,
$R^{12}$ represents a hydrogen atom or $COR^{15}$,
$R^{13}$ and $R^{14}$ each represent a hydrogen atom or alkyl group of form 1 to 4 carbon atom(s) or $NR^{13}R^{14}$ represents an amino acid residue or heterocyclic ring, or
$R^{15}$ represents an alkyl group of from 1 to 4 carbon atom(s) or phenyl group,

EP 0 516 181 A1

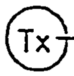 represents (i) 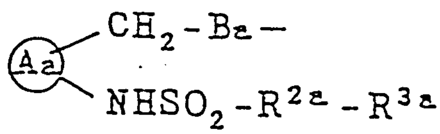 (A)

wherein

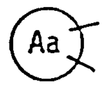

represents

(i) 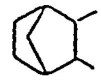 (Aa-1),

(ii) 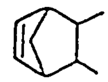 (Aa-2),

(iii) 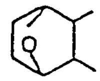 (Aa-3),

(iV) 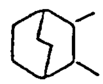 (Aa-4),

(V) 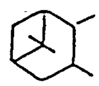 (Aa-5),

(Vi) 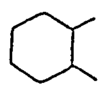 (Aa-6),

(Vii) 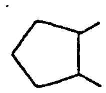 (Aa-7),

(Viii) 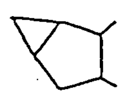 (Aa-8),

(iX) 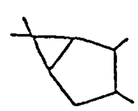 (Aa-9),

(X) 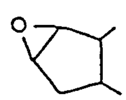 (Aa-10),

(Xi) 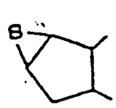 (Aa-11),

Ba represents

(i) $-CH_2-CH_2-(CH_2)m-$ (Ba-1),

(ii) cis-CH = CH-$(CH_2)$m- (Ba-2),
(iii) -$CH_2$-O-$(CH_2)$m- (Ba-3),
(iV) -S-$(CH_2)$m- (Ba-4) or
(V)

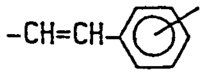

(Ba-5)

wherein m represents an integer of from 1 to 6, and the configuration of a double bond is E, Z or EZ and a phenylene group represetns an o-, m- or p-phenylene group in the formula (Ba-5),

$R^{2a}$ represents a bond or an alkylene group of from 1 to 4 carbon atom(s),

$R^{3a}$ represents a carbocyclic or heterocyclic ring unsubstituted or substituted by from one to three of an alkyl group of from 1 to 4 carbon atom(s), alkoxy group of from 1 to 4 carbon atom(s), hydroxy group, carboxyl group, ciano group, halogen atom or nitro group, or

$R^{2a}$ represents an alkyl group of from 1 to 12 carbon atom(s) together with $R^{3a,}$

cyclodextrin clathrates thereof, or non-toxic salts thereof in case that $NR^{13}R^{14}$ represents an amino acid residue, possess an antagonistic activity on $TXA_2$, in particular, inhibit blood platelet aggregation and contraction of artery, and are, therefore, useful for prevention and/or treatment of inflammation, hypertension, thrombus, cerebral apoplexy, asthma, cardiac infarction, angina pectoris, cerebral infarction and death by aucte cardiac diseases in mammales, in particular in humans, which are induced by thromboxane $A_2$.

Summary

This invention is related to novel sulfonamide derivatives.

More particularly, this invention is related to

(1) sulfonamide derivatives of the general formula

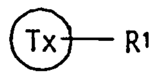

(I)

(wherein, all of the symbols represent the same meaning as hereinafter defined.),

(2) process for the preparation of them and

(3) treating agent containing them as active ingredient.

Background

In 1975, Hamberg et al discovered an unstable intermediate in the conversion of prostaglandin G2 into the hemiacetal derivative in platelets [Proc. Nat. Acad. Sci. U.S.A., Vol 72, No.8 page 2994 (1975)]. The intermediates have been named as thromboxane $A_2$ and its structure has been proposed as follows:

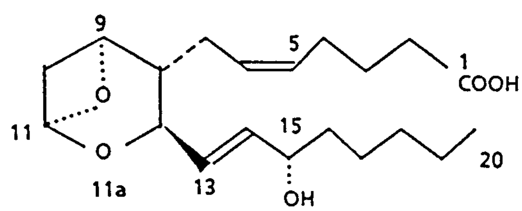

(a)

$TXA_2$ has been found to show various biological activities such as platelet aggregation, aorta contraction and thrombi formation and therefore is considered to be one of the cause by which diseases such as inflammation, thrombus and cardiac infarction are induced.

Some $TXA_2$ analogues are proposed as compounds having antagonistic activity on $TXA_2$; for example, compounds which replaced the oxygen atoms on 11a- and 9,11-epoxy-position of $TXA_2$ by carbon atoms [see Japanese Patent Kokai No. 55-143930], and compounds having pinane skelton [Proc. Nat. Acad. Sci. U.S.A. Vol. 76, No. 6, page 2566 (1979)]. And, the compounds described below which have a cyclopentane skeleton are also known.

(±) 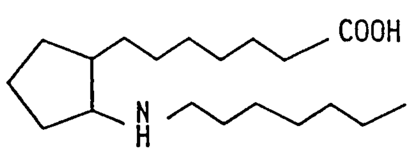 (b)

More recently, in the specification of GB No.2184118, the carbocyclic sulfonamide derivatives of the general formula:

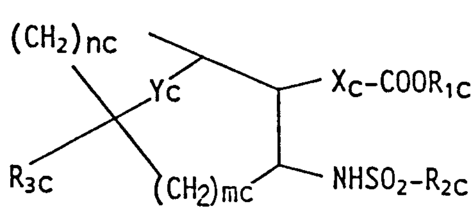

(c)

(wherein $R_{1C}$ represents a hydrogen atom or lower alkyl group,

$R_{2C}$ represents an alkyl group, substituted or unsubstituted aryl group, aralkyl group or heretocyclic ring,

$R_{3C}$ represents a hydrogen atom or methyl group,

Xc represents a alkylene group or alkenylene group substituted by one or more fluoro atom(s) or, containing

one oxygen atom, sulfur atom or phenylene group thereinto,
Yc represents a straight-chain or branched-chain alkylene group or alkenylene group, oxygen atom or sulfur atom,
mc represents an integer of 0 or 1, and
nc represents an integer of 0, 1 or 2.) have been disclosed. And, the following compound was published by Bayer A.G. at 16th International Symposium on the Chemistry of Naitonal Products opened on May 29 - June 3, 1988.

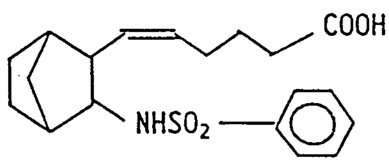

(d)

Brief description of drawings

Fig. 1, Fig. 2 and Fig. 3 show the change of rate which the compounds of the present invention inhibit increasing of blood pressure induced by $STA_2$ in guinea pig at time elapsed.

Disclosure of the invention

The present invention is related to sulfonamide derivatives of the general formula:

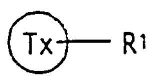

(I)

wherein, $R^1$ represents
(i) $COOR^{11}$,
(ii) $CH_2OR^{12}$ or
(iii) $CONR^{13}R^{14}$, wherein
$R^{11}$ represents a carbocyclic ring unsubstituted or substituted by an alkyl or alkoxy group of from 1 to 4 carbon atom(s) or halogen atom, or steroid,
$R^{12}$ represents a hydrogen atom or $COR^{15}$,
$R^{13}$ and $R^{14}$ each represent a hydrogen atom or alkyl group of form 1 to 4 carbon atom(s) or
$NR^{13}R^{14}$ represents an amino acid residue or heterocyclic ring, or
$R^{15}$ represents an alkyl group of from 1 to 4 carbon atom(s) or phenyl group,

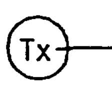 represents 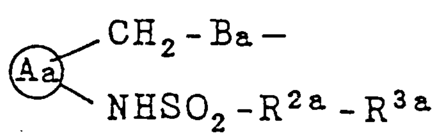

(A)

wherein

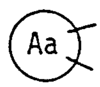

represents

(i) 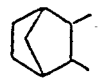 (Aa-1),

(ii) 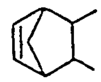 (Aa-2),

(iii) 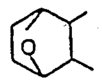 (Aa-3),

(iV) 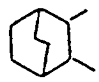 (Aa-4),

(V) 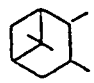 (Aa-5),

(Vi) 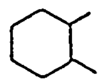 (Aa-6),

(Vii) 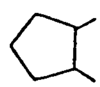 (Aa-7),

(Viii) 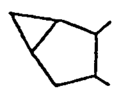 (Aa-8),

(iX) 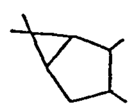 (Aa-9),

(X) 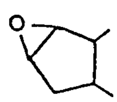 (Aa-10),

(Xi) 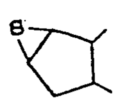 (Aa-11),

Ba represents

(i) $-CH_2-CH_2-(CH_2)m-$ (Ba-1),

(ii) cis-$CH=CH-(CH_2)m-$ (Ba-2),

(iii) $-CH_2-O-(CH_2)m-$ (Ba-3),

(iV) -S-$(CH_2)$m- (Ba-4) or
(V)

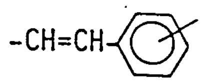 (Ba-5)

wherein m represents an integer of from 1 to 6, and the configuration of a double bond is E, Z or EZ and a phenylene group represents an o-, m- or p-phenylene group in the formula (Ba-5),

$R^{2a}$ represents a bond or an alkylene group of from 1 to 4 carbon atom(s),

$R^{3a}$ represents a carbocyclic or heterocyclic ring unsubstituted or substituted by from one to three of an alkyl group of from 1 to 4 carbon atom(s), alkoxy group of from 1 to 4 carbon atom(s), hydroxy group, carboxyl group, ciano group, halogen atom or nitro group, or

$R^{2a}$ represents an alkyl group of from 1 to 12 carbon atom(s) together with $R^{3a}$;

cyclodextrin clathrates thereof, or non-toxic salts thereof in case that $NR^{13}R^{14}$ represents an amino acid residue, process for the preparation of them, and treating agent containing them as active ingredient.

The terms of alkyl group, alkylene group, alkenylene group and alkoxy group in description each symbol throughout the present specification including claims mean straight-chain or branched-chain alkyl group, alkylene group, alkenylene group and alkoxy group.

The configuration of double bonds in alkenylene groups are E, Z and E, Z mixtures. Isomers generated by asymmetric carbons existing in case that branched alkyl group are also included.

The presence of asymmetric centers leads, as is well known, to the existence of isomers. And all each optical isomers and all mixtures thereof are included in the general formula (1). For instance, a mixture of one optical isomer and enantiomer thereof, a racemic body which is an equivalent mixture especially and a mixture of one optical isomer and diastereomer thereof are also included.

In the structural formulae throughout the present specification dashed line (-----) indicate α-configuration tapered line

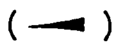

indicate β-configuration, wavy line

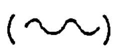

indicate α- or β- configuration or mixture thereof.

In the general formula (I), the ring structures of the formula

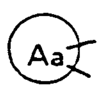

are named each and numbered at each position as follows.

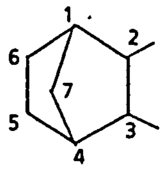
,

(Aa-1)

bicyclo[2.2.1]
heptane

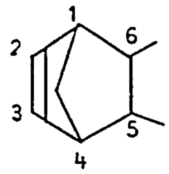
,

(Aa-2)

bicyclo[2.2.1]
hept-2-ene

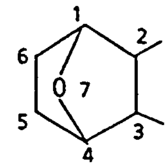
,

(Aa-3)

7-oxabicyclo
[2.2.1]heptane

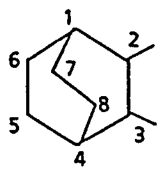
,

(Aa-4)

bicyclo[2.2.2]
octane

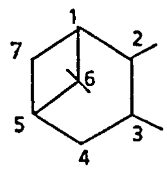
,

(Aa-5)

6,6-dimethyl
bicyclo[3.1.1]
heptane

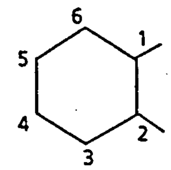
,

(Aa-6)

cyclohexane

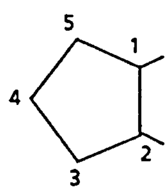
,

(Aa-7)

cyclopentane

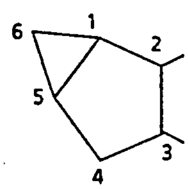
,

(Aa-8)

bicyclo[3.1.0]
hexane

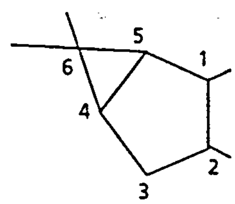
,

(Aa-9)

6,6-dimethyl
bicyclo[3.1.0]
hexane

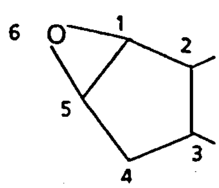
,

(Aa-10)

6-oxa-bicyclo
[3.1.0]hexane

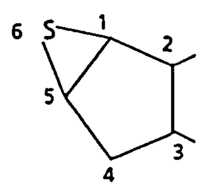

(Aa-11)

6-thiobicyclo
[3.1.0]hexane

As will be apparent to those skilled in the art, ring structures described above have two [in case of the formulae (Aa-4), (Aa-6) and (Aa-7)] or four [in case of the formulae (Aa-1), (Aa-2), (Aa-3), (Aa-5), (Aa-8), (Aa-9), (Aa-10) and (Aa-11)] asymmetric carbon atoms. Namely they are 1-, 2-, 3- and 4- position carbon atoms in the formulae (Aa-1), (Aa-2) and (Aa-3), 2- and 3- position carbon atoms in the formulae (Aa-4), 1 and 2- position carbon atoms in the formulae (Aa-6) and (Aa-7) and 1-, 2-, 3- and 5- position carbon atoms in the formulae (Aa-5), (Aa-8), (Aa-9), (Aa-10) and (Aa-11).

The presence of asymmetric centers leads, as is well known, to the existence of isomers. And all each optical isomers and all mixtures thereof are included in the general formula (I). For instance, a mixture of

one optical isomer and enantiomer thereof, a racemic body which is an equivalent mixture especially and a mixture of one optical isomer and diastereomer thereof are also included.

In the general formula (I) steroids shown by $R^{11}$ are the compounds which have steroid skeleton substituted by various substituents, for example and preferably cholesterol.

Carbocyclic ring in the group shown by $R^{11}$, are mono-, bi- or tri- aromatic carbocylic rings containing not more than 15 carbon atoms which may be partially or fully saturated.

The rings are, for example, benzene, naphthalene, indene, aqulene, fluorene, phenanthrene, anthracene, acenaphthylene, biphenylene ring and the rings which may be partially or fully saturated thereof. Preferable rings are benzene and cyclohexane. Alkyl groups of from 1 to 4 carbon atom(s) shown by substituents in groups which $R^{11}$ represent are methyl, ethyl, propyl, butyl and isomeric groups thereof and alkoxy groups of from 1 to 4 carbon atom(s) are methoxy, ethoxy, propoxy, butoxy and isomeric group thereof and halogen atom are fluorine, chlorine, iodine and bromine atom. Preferable substituents in $R^{11}$ are methyl and isopropyl group. Carbocyclic rings in $R^{11}$ are also preferable to be unsubstituted.

In general formula (I), preferable $R^{12}$ are hydrogen atom, acetyl and benzoyl gorup.

In general formula (I), amino acid residues shown by $NR^{13}R^{14}$ mean $\alpha$-amino acid residues wherein a hydrogen atom in the amino group is removed.

For example, they are glycine, alanine, valine, isoleucine, leucine, serin, threonine, proline, asparagine, glutamine, methionine, phenylalanine, tyrosine, aspartic acid, glutamic acid and lysine residue.

In the general formula (I), heretocyclic rings shown by $NR^{13}R^{14}$ are mono-, bi- or tri- aromatic heterocyclic rings containing not more than 15 carbon and hetero atoms which may be partially or fully saturated. For example, they are pyrrole, oxazole, imidazole, pyrazole, pyridine, pyrimidine, pyridazine, pyrimidine, pyrazine, pyrroline, pyrrolidine, imidazolidine, pyrrolidine is preferable.

Preferable ring structures shown by

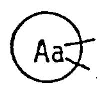

are the bridged ring shown by the formula (Aa-1), (Aa-2), (Aa-3), (Aa-4) and (Aa-5). Especially preferable group is a bridged carbocyclic ring shown by the formula (Aa-1).

In the general formula (I), out of groups represented by Ba, groups of the formulal (Ba-2) and (Ba-5), having a double bond are prefered. Especially preferable group is a group shown by the formula (Ba-2).

In the general formula (I), groups shown by the formula $-(CH_2)m-$ represent methylene, ethylene, trimethylene, tetramethylene, pentamethylene and hexamethylene group. Preferable group is a trimethylene group.

In the general fromula (I), groups shown by $R^{2a}$ represent a bond and an alkylene group of from 1 to 4 carbon atom(s). Preferable group is a bond.

In the general formula (I), groups shown by $R^{3a}$ represent carbocyclic and heterocyclic rings unsubstituted and substituted by alkyl group of from 1 to 4 carbon atom(s), alkoxy gorup of from 1 to 4 carbon atom(s), nitro group, hydroxy group, carboxyl group, ciano group or halogen atom. Carbocyclic rings described herein are mono-, bi- or tri- aromatic carbocyclic rings containing not more than 15 carbon atoms which may be partially or fully saturated. heterocyclic rings described herein are mono-, bi- or tri- aromatic heterocyclic rings containing not more than 15 carbon and hetero atoms which may be partially or fully saturated. For example they are benzene, naphthalene, pyridine ring. Benzene ring is preferable. Especially preferable $R^3$ is 4-methylphenyl group.

Alkyl groups of from 1 to 12 carbon atom(s) which $R^{2a}$ and $R^{3a}$ represent together, are methyl, ethyl, propyl, butyl, pentyl, hexyl, heptyl, octyl, nonyl, decyl, undecyl, dodecyl and isomeric groups thereof. All groups are preferable.

## Cyclodextrin Clathrates

The cyclodextrin clathrates of the compounds shown by the general formula(I), of the present invention may be prepared with using $\alpha$-, $\beta$- or $\gamma$- cyclodextrin or a mixture thereof, by the method described in the specification of British Patent Nos. 1,351,238 or 1,419,221.

By converting into cyclodextrin clathrates, the stability of the compounds of the formula(I) can be increased.

## Salts

The compounds in which $NR^{13}R^{14}$ represents an amino acid among the compounds of the general formula(I), of the present invention may be converted into the corresponding salts. Non-toxic and water-soluble salts are preferable. Suitable salts, for example, are follows:
salts of alkaline metal (sodium, potassium etc.),
salts of alkaline earth metal (calcium, magnesium etc.),
ammonium salts, salts of pharmaceutically acceptable organic amine (tetramethylammonium, triethylamine, methylamine, dimethylamine, cyclopentylamine, benzylamine, phenethylamine, piperidineamine, monoethanolamine, diethanolamine, tris(hydroxymethyl)amine, lysine, arginine, N-methyl-D-glucamine etc.).

Comparison with prior arts

The compounds of the general formula(I), of the present invention are perfectly novel compounds which have quite different chemical structures from prior known compounds.

Described concretely,

The compounds in which

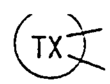

represents the formula(A) are carboxylic acid derivatives such as esters with carbocyclic rings and steroids, alcohols and amides with amino acids and heterocyclic ring in the present invention, though the prior compounds inwhich represents the formula(A) are only carboxylic acids and alkyl ester.

Accordingly, the compounds of the present invention are novel compounds having diferent structures from the prior compounds. It can be unexpected that the compounds which have different structures from the prior $TXA_2$ antagonists as described above, also possess an antagonistic effect against $TXA_2$.

Moreover, the compounds of the general formula(I), of this invention possess more useful features in pharmacodynamic effect than the prior $TXA_2$ antagonists.

Described concretely, the compounds of the present invention possess an antagonistic activity against $TXA_2$ and a part of them possess significantly stronger activity than the prior compounds of the formula(b) and(c). And, the inventors have confirmed that some compounds of the present invention hardly possess a side effect i.e. an agonistic activity against $TXA_2$ (effect of increasing of blood pressure) which the prior $TXA_2$ antagonists possess. It could be unexpected that the compounds prepared by changing the structures of the prior $TXA_2$ antagonists as described above possess different activities from the prior $TXA_2$ antagonists, until the inventors synthesized them and confirmed their activities.

Process for the preparation for the compounds of the present invention.

According to the present invention, the compounds of the general formula (I), of the present ivnention may be prepared by the step described hereinafter.

Step 1:

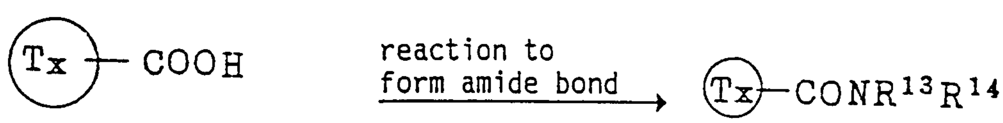


(Ib) (Ic)

Step 2:

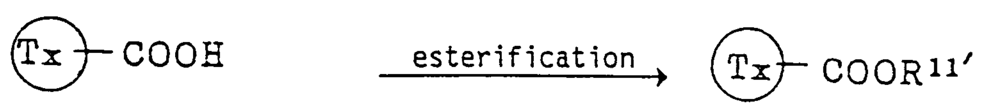


(Ib) (Id)

Step 3:

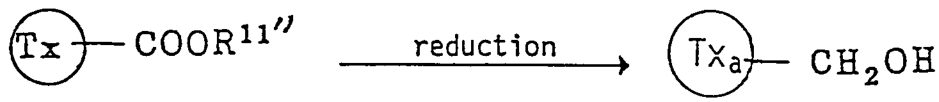


(Id')  (Ie)

Step 4:

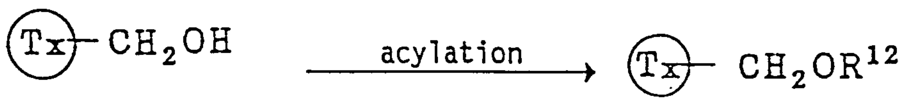


(Ie)  (If)

$R^{11'}$ represents an alkyl group of from 1 to 20 carbon atom(s), carbocyclic ring unsubstituted or substituted by an alkyl or alkoxy group of from 1 to 4 carbon atom(s) or halogen atom, or steroid, $R^{11''}$ represents an alkyl group of 1 to 8 carbon atom(s) and the other symbols represent the same meaning as described hereinbefore.

Step 1 is the reaction to form amide bond. For example, the reaction may be carried out by reacting (i) with using a corresponding amine shown by the formula $H_2NR^{13}$ ($R^{13}$ represents the same meaning as described hereinbefore), in an inert organic solvent (methylene chloride, toluene etc), at a temperature of from 0 °C to 40 °C, after reacting oxalyl chloride, (ii) with using a corresponding amine shown by the formula $H_2NR^{13}$ ($R^{13}$ represeents the same meaning as described hereinbefore), e.g. 2-chloro-1-methylpyridinium iodide and tertiary amine (triethylmaine etc), in an inert organic solvent (methylene chrolide etc), at a temperature of from 0 °C to 40 °C. In case that an amine shown by the formula $HNR^{13}R^{14}$ represents an amino acid, the reaction is carried out by reacting the compounds in which a carboxyl group in an amino acid is protected by an appropriate alkyl group, or the compounds in which an amino group having no connection to the reaction is protected by a tert-butoxycarbonyl group (boc group) or a benzyloxy carbonyl group (cbz group), and then hydrolyzing with using an acid (trifluoroacetic acid etc.) or an alkali (sodium hydroxide etc.) to remove a protecting group.

Step 2 is esterification. For example, it may be carried out by (i) using the corresponding diazoalkane in an inert organic solvent (diethylether, methylene chloride etc) at a temperature of from 0 °C to 40 °C, (ii) using the corresponding alkylhalide in the presence of a base (sodium carbonate etc.) in an inert organic solvent (acetone, dimethylsulfoxide etc.) at a temperature of from -10 °C to 80 °C, or (iii) reacting an acid chloride corresponding to the acid of the formula (Ib) and the desired alcohol at a temperature of from -10 °C to 40 °C.

Step 3 is reduction. It may be carried out by reacting with using lithium aluminum hydride or diisobutyl aluminum hydride, in an inert organic solvent (tetrahydrofuran, diethylether, lower alkanol etc.), at from -78 °C to a room temperature.

Step 4 is acylation. It may be carried out by reacting an acylhalide or acid anhydride in the presence of a tertiary amine (pyridine, triethylamine) in an inert organic solvent (diethylether, tetrahydrofuran, methylene chloride) or no solvent at a temperature of from -20 °C to 50 °C.

Process for the preparation of starting material

The compounds of the general formula:

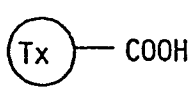

(Ib-1)

(wherein

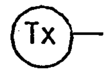

represents the formula (A).) in the compounds used as the starting materials, are described in the specification of British Patent Publication No.2184118 and they may be prepared by the following method.

Step 1'-1:

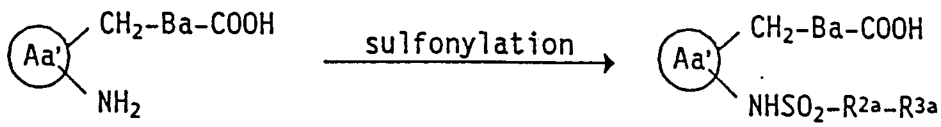


(V) (Ib-1'''')

Step 1'-2:

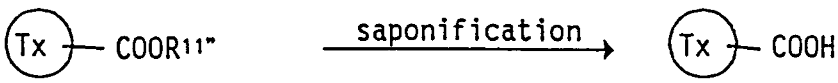


(Id-1) (Ib-1)

Step 2':

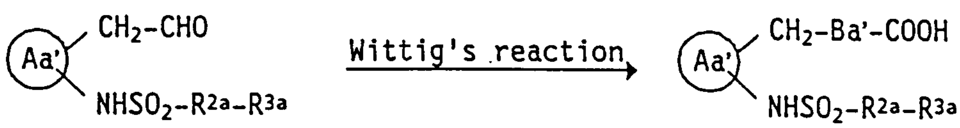


(VI) (Ib-1')

Step 3':

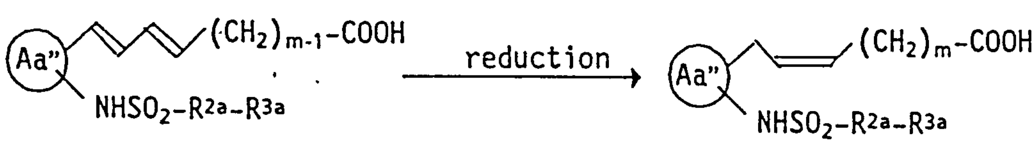


(VII) (Ib-1'')

Step 4':

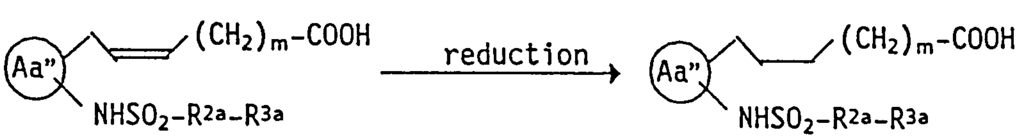


(Ib-1'') (Ib-1''')

(wherein

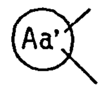

represents a group of the fomulae (Aa-1), (Aa-2), (Aa-3), (Aa-4), (Aa-5), (Aa-6) or (Aa-7),

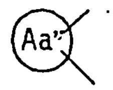

represents a group of the formulae (Aa-1), (Aa-3), (Aa-4), (Aa-6) or (Aa-7),
Ba' represents a group of the formulae (Ba-2) or (Ba-5),
the other symbols represent the same meaning as described hereinbefore, with the proviso that the configurations of two double bond in formula (5) are each E, Z or EZ.).

Explained each reaction steps in detail,

Step 1'-1 is sulfonylation reaction. For example, it may be carried out with using a sulfonyl halide of the formula:

$X\text{-}SO_2\text{-}R^{2a}\text{-}R^{3a}$ (2)

(wherein, X represents a halogen atom and the other symbols represent the same meanings as defined hereinbefore.)
in the presence of Lewis' base (tertiary amine such as triethylamine, pyridine, dimethylaminopyridine etc) as a stimulator, in an inert organic solvent (methylene chloride etc), at a temperature of from -40 °C to 50 °C (preferably, at from 0 °C to room temperature).

Step 1'-2 is saponification. For example, it is carried out with using an aqueous solution of an alkali (sodium hydroxide, potassium carbonate, lithium hydroxide etc.), in a water-soluble organic solvent (tetrahydrofuran, methanol, ethanol etc.) usually at a temperature of from -10 °C to 100 °C.

Step 2' is well known reaction as Wittig's reaction. For example, it may be carried out by reacting an aldehyde of the general formula (IV)and a phosphonium salt of the general formula:

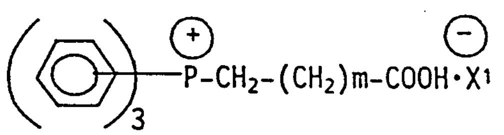
(3) or

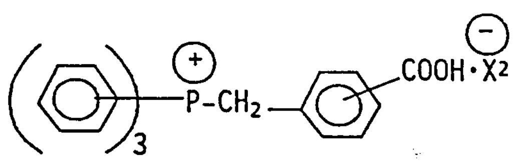
(4)

(wherein, $X^1$ and $X^2$ each represent a halogen atom and m represents the same meaning as defined hereinbefore)
in the presence of a strong base (potassium tert-butoxide, lithium diisopropylamide, sodium hydride etc), in an inert organic solvent (toluene, tetrahydrofuran, dimethylsulfoxide etc.), at from -78 °C to a room temperature.

Step 3' may be carried out by reacting with using methyl benzoate chromium tricarbonyl, as catalyst, in an inert organic solvent (acetone etc.), in an atmosphere of hydrogen, at from 70 to 120 atmospheres, at from 100 °C to 150 °C.

Step 4' is reduction reaction. For example, it may be carried out in the presence of hydrogenation catalyst (palladium-carbon, nickel etc), in an alkanol, in an atmosphere of hydrogen, usually at ordinary temperature and pressure or by raising a little the temperature and atmosphere as occasion demands.

The compounds of the formulae (V), (VI) and (VII) using as starting materials partially include known compounds. New compounds of the present inventional also may be prepared by known method. For example, the compounds of the formula (V) and (VI) wherein represents the formula (Aa-5) may be prepared

as follows. And the process of preparation for a part of the compounds shown by the formula (V) was described in the specification of Japanese Patent Kokai No. 61-49, i.e European Patent Publication No. 0171146.

In the following formulae, THP represents tetrahydropyran-2-yl group and the other groups represent the same meaning as defined hereinbefore.

Scheme [A]

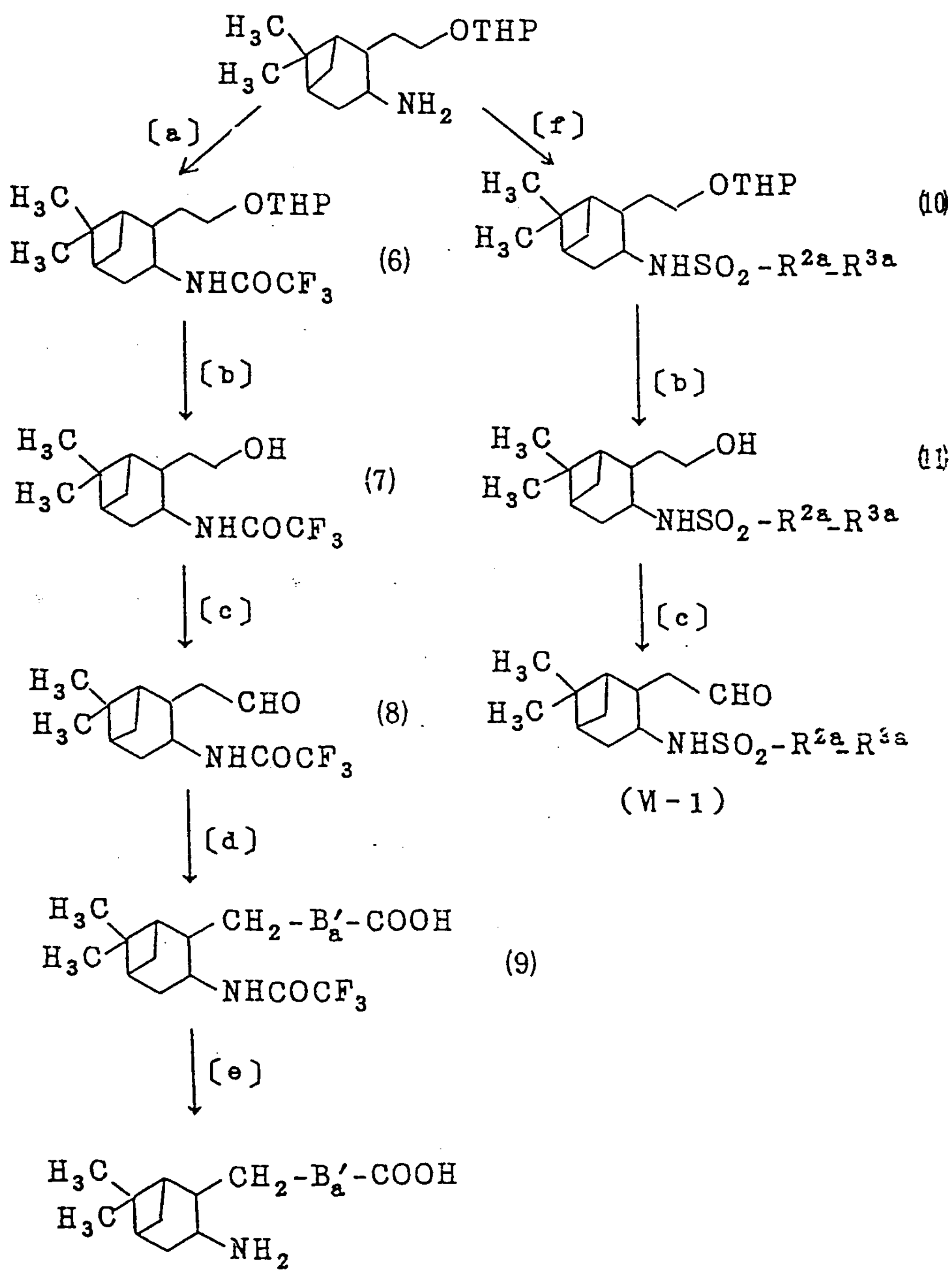


(6) (10) (7) (11) (8) (VI-1) (9)

(V-1)

Explained each steps briefly, step [a] is the introduction of a protecting group (trifluoroacetyl group) into

an amino group. For example, it may be carried out with using anhydrous trifluoroacetic acid, in the presence of a tartiary amine (pyridine etc.), in an inert organic solvent (methylene chloride etc.).

Step [b] is elimination reaction of protecting group of hydroxyl group. For example, it may be carried out with using an acid (p-toluene sulfonic acid, acetic acid etc.), in an organic soluvent (methanol-water etc.)

Step [c] is oxidation. For example, it may be carried out with using an oxidizing agent (oxalyl chloride-dimethylsulfoxide, sulfur trioxide-pyridine complex, dimethylsulfoxide etc), in the presence of a tertiary amine (triethylamine etc.), in a suitable organic solvent (metylene chloride, dimethylsulfoxide etc.).

Step [d] is wittig's reaction. It may be carried out with using a phosphorane compound of the formula (3) or (4), by the same procedure as described in step 2'.

Step [e] is saponification. For example, it is carried out (i) with using an aqueous solution of an alkali (sodium hydroxide, potassium carbonate, lithium hydroxide etc.), in a water - soluble organic solvent (tetrahydrofuran, methanol, ethanol etc.), or (ii) with using an alkali described hereinbefore, under the anhydrous condition, in an alkanol (methanol, ethanol etc). These reactions may be carried out usually at a temperature of from -10 ° C to 100 ° C.

Step [f] is sulfonylation reaction. It may be carried out by the same procedure as described step 1'-1.

The compound of the formula (5) may be prepared as follows.

Scheme [B]

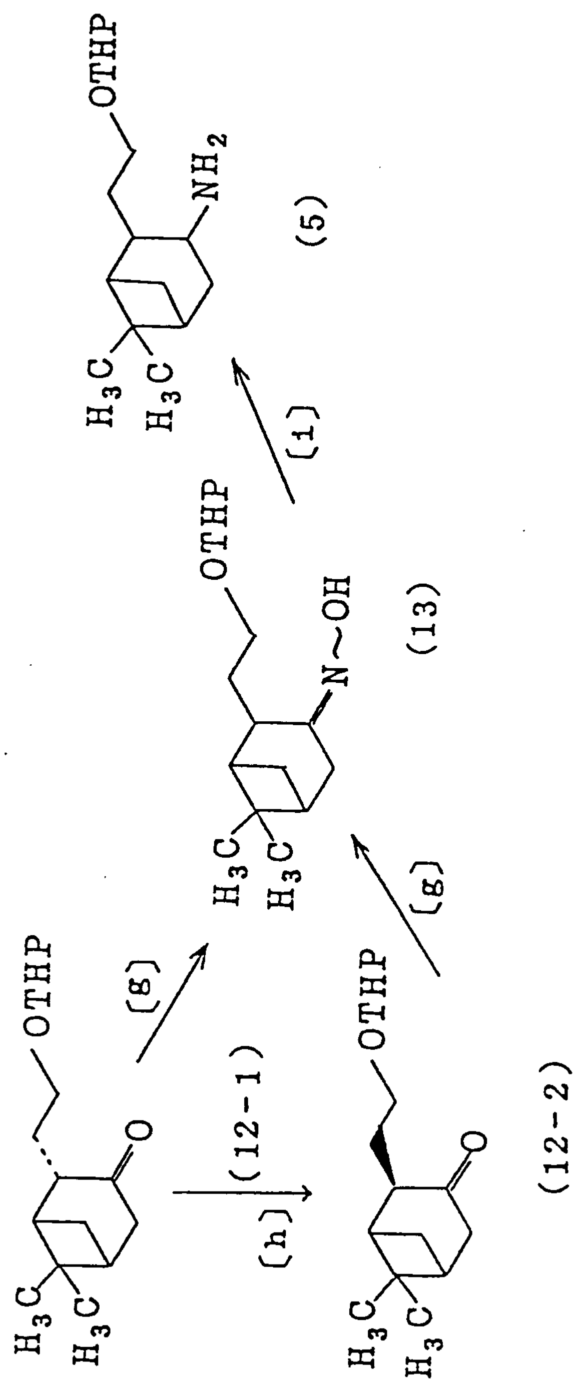


(12-1) (12-2) (13) (5)

Explained each steps briefly, step [g] is the reaction to form oxime. It may be carried out by reacting with using a hydroxylamine hydrochloride, in the presence of barium carbonate, in an inert organic solvent (lower alkanol etc.) at from a room temperature to a refluxing temperature.

Step [h] is inversion. It may be carried out by refluxing with using a base (potassium tert-butoxide etc.), in an inert organic solvent (tetrahydrofuran, lower alkanol etc.).

Step [i] may be carried out by reducing the compound of the formula (13) with using Raney Nickel as catalyst, or refluxing with using metallic sodium in an lower alkanol (propanol etc.).

The compund of the formula (12-1) may be prepared with using $\beta$-pinene, $\alpha$-pinene and 2-oxonorpinane as starting material by the same process as described in the specification of Japanese Patent Kokai No. 61-49.

The compounds of the fomula (V), (VI) and (VII) wherein

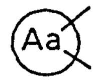

represents the groups of the formula (Aa-1), (Aa-3), (Aa-4), (Aa-6) and (Aa-7) may be prepared, for example, as follows. In scheme, $R^{5'}$ represents a phenyl group substituted or unsubstituted, or lower alkyl group. The other symbols represent the same meaning as defined hereinbefore.

Scheme [C]

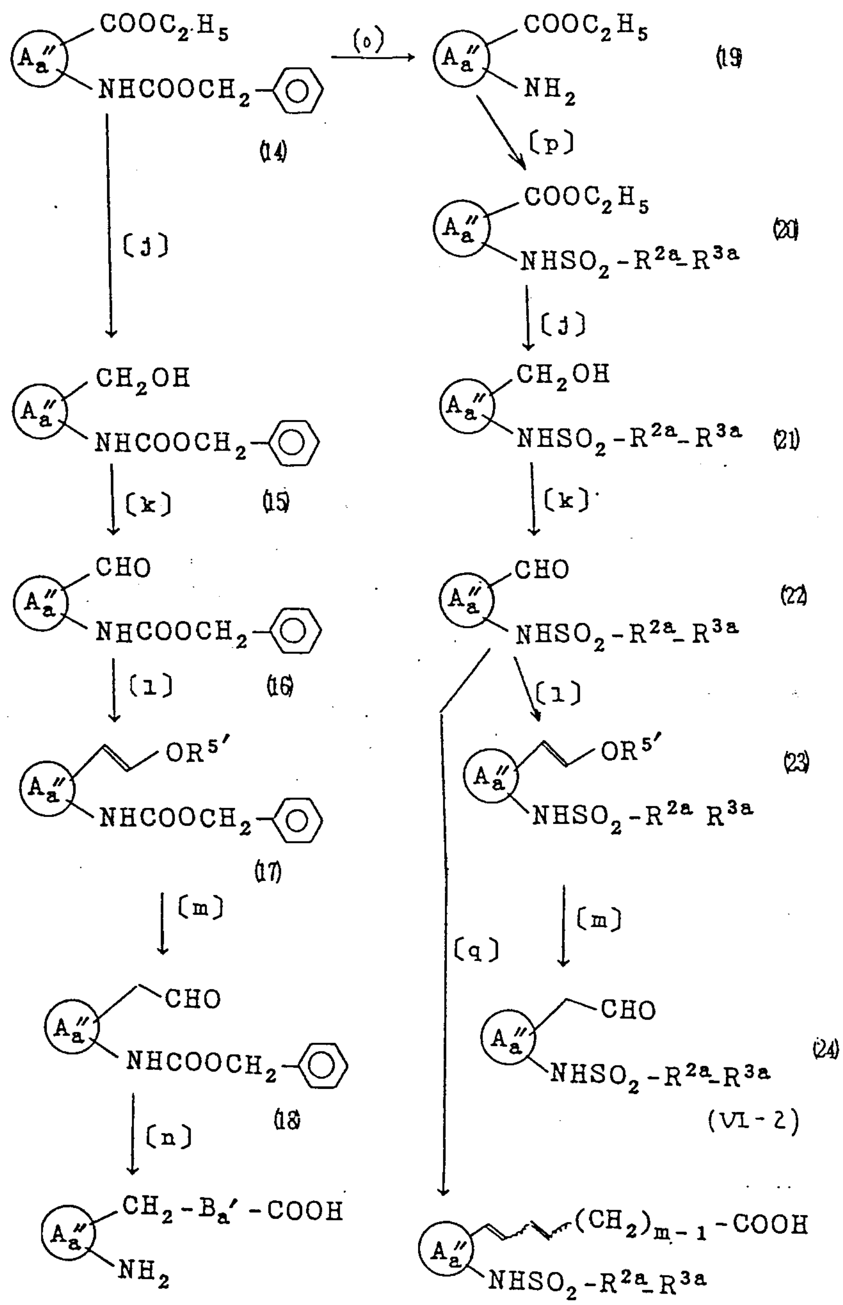


(V-2) (VII-1)

Explained each steps briefly, step [j] is reduction. It may be carried out with using lithium aluminum hydride or diisobutyl aluminum hydride, in an inert organic solvent (tetrahydrofuran, diethylether, lower alkanol etc.), at from -78 ° C to a room temperature.

Step [k] is oxidation. It may be carried out by the same procedure as step [c] described hereinbefore.

Step [l] is wittig's reaction. It may be carried out with using the compound of the general formula:

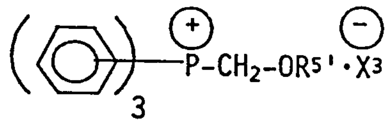

(25)

(wherein, $R^{5'}$ represents the same meaning defined hereinbefore $X^3$ represents a halogen atom.), under the same condition as step [d] descrivbed hereinbefore.

Step [m] is hydrolysis. It may be carried out by reacting with using an acid (hydrochloric acid, acetic acid, oxalic acid etc.), in an inert organic solvent (THF, dioxane, lower alkanol etc.), at a temperature of from 50 ° C to 100 ° C.

Step [n] may be carried out by the same procedure as from step [d] to [e].

Step [o] is elimination of a benzyloxycarbonyl group. It may be carried out by the same reduction as step 4 described hereinbefore.

Step [p] is sulfonylation. It may be carried out by the same procedure as step 1 described hereinbefore.

Step [q] is wittig's reaction. It may be carried out with using the compound of the general formula:

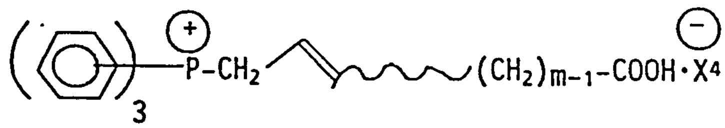

(26)

(wherein $X^4$ represents a halogen atom, m represents the same meaning as described hereinbefore.) under the same condition as step [d] described hereinbefore.

The compound of the formula (14) may be prepared as follows.

Scheme [D]

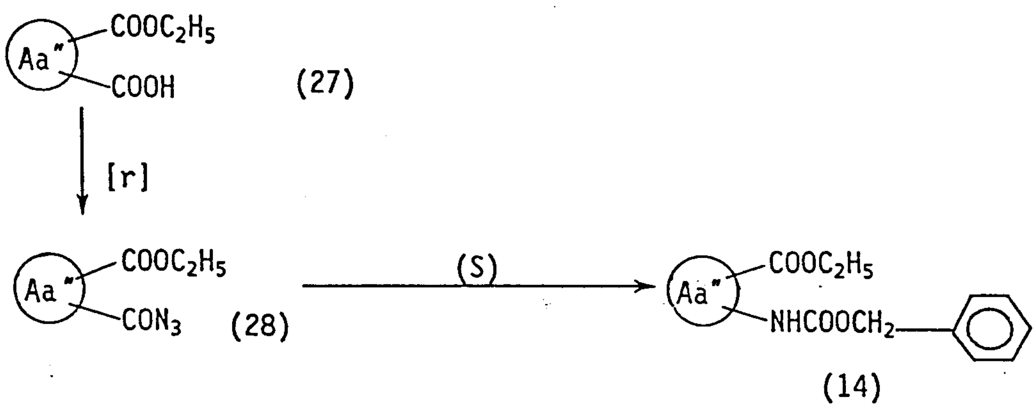


(27) (28) (14)

Explained each steps briefly, step [r] may be carried out by reacting with using an ethyl chloroformate, in the presence of a tertiary amine (triethylamine etc.), in an inert organic solvent (acetone etc.), at from -10 ° C to a room temperature, then adding sodium azide and reacting at the same temperature as above.

Step [s] may be carried out by refluxing the compound of the formula (28) in an inert organic solvent (toluene etc.) to obtain the corresponding isocyanate and then reacting it and benzyl alcohol in the presence of a tertiary amine (triethylamine etc.) or an acid (p-toluenesulfonic acid etc.).

The compound of the formula (27) used as starting material may be prepared by

(1) refluxing the corresponding acid anhydride in ethanol,

(2) hydrolyzing the corresponding diethyl ester by one equivalent of alkali or

(3) hydrolyzing the corresponding diethyl ester with using a hydrolytic enzyme.

Acid anhydrides and diethyl esters corresponding to the compound of the formula (27) are known as (±) bodies or may be prepared easily from known dicarboxylic acids.

If desired, (±) bodies may be resolved into each optically active compounds. The optical resolution may be carried out by the known method [See Tables of resolving agents and optical resolutions, University of Hotre dame press (1972) etc.] in a preferable step, for example, step of the compound (27).

Optical active compounds of the compounds of the general formula (I), wherein

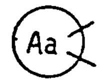

represents a group of the formula (Aa-1), (Aa-3), (Aa-4) or (Aa-6) and which have such a configuration that the upper chain ($-CH_2-Ba-R^1$) and the lower chain ($-NHSO_2-R^{2a}-R^{3a}$) bond in trans against the ring with respect to each other, may be prepared as follows: 1,3-cyclopentadiene, 1,3-cyclohexadiene, furan or butadiene are reacted with dimenthyl fumarate or menthylmandelyl fumarate in the presence of Lewis' acid. The optically active Diels-Alder adduct thus obtained may be converted into a monomenthylester carboxylic acid or the other monoalkylester carboxylic acid, corresponding to the compound of the formula (27) to subject subsequent reactions. On this occasion, a double bond in the Diels - Alder adduct must be reduced in a suitable step [for example, the Diels-Alder adduct or the compound of the fomrula (19)].

Each starting materials and reagents, used in the present invention are known per se or may be prepared by methods known per se.

Throughout the specification, in each reactions, products may be purified by conventional methods, for example, distillation at atmospheric or reduced pressure, high performance liquid chromatography, thin layer chromatography or column chromatography using silica gel or magnesium silicate or washing or recrystallization. Purification may be carried out after each reactions or a series of reactions.

[Cyclodextrin clathrates and salts]

The cyclodextrin clathrates of carbocyclic sulfonamide derivatives shown by the general formula (I) may be prepared with using $\alpha$-, $\beta$- or $\gamma$- cyclodextrin or a mixture thereof, by the method described in the specification of Japanese Patent Kokoku Nos. 50-3362 or 52-31404 and British Patent No. 1351238 and U.S. Patnet No. 3816393.

The stabilities of the compounds shown by the general formula (I) are enlarged by converting them into cyclodextrin clathrates.

The acid of the general formula (I) wherein $NR^{13}R^{14}$ represents an amino acid residue, are converted into salts by known methods.

The salts are preferably non-toxic ones. The non-toxic salts herein referred mean salts of cations such that it is relatively innoxious to living body (animals including humans) tissues and that the effective pharmacological properties of the compound(s) of the general formula (I) are not impaired by side effect(s) resulting from the cations when used in an amount required for the treatment.

And water-soluble salts are preferable.

Suitable salts include, for example, a salt of an alkali metal (sodium, potassium etc.), a salt of an alkaline earth metal (calcium, magnesium etc.), an ammonium salt and a pharmaceutically acceptable (non-toxic) amine salt.

Amines suitable for forming such salts with carboxylic acid are well known, and include, for example, those amines which are theoretically obtained by substituting one or more of hydrogen atom(s) of ammonia by other groups.

Examples of such amine are, an amino acid (arginine, lysine, glutamine, histidine, asparagine etc), a sugar-amine (N-methylglucane, N-methylmannosamine, N-methylgalactosamine, N-methylfructosamine, N-methylarabinosamine, N-methylribosamine, N-methyllactosamine etc.) and another amine (ethanolamine, triethanolamine, triethylamine, meglumine, guanidine etc.).

The salt can be obtained by known method par se, for example, by reacting an acid of the general formula (I) wherein $NR^{13}R^{14}$ represents an amino acid residue, with a suitable base (e.g. a hydroxide or carbonate of an alkali metal or an alkaline earth metal, ammonia or an amine) in theoretical amounts in an appropriate solvent.

The salt can be isolated by freeze-drying the solution, or by filtration if the salt is sufficiently insoluble to the reaction solution, or if necessary, by removing part of the solvent followed by filtration.

[Pharmacological Activities of the compounds of the present invention]

The compounds of the general formula (I), of the present invention, possess an antagonistic action against thromboxane $A_2$, especially inhibitory effect on platelet aggregation, on contraction of smooth muscle or on increasing of blood pressure.

For example, in standard laboratory test, the effects were possible to be confirmed by (i) inhibitory effect on contraction of smooth muscle induced by $STA_2$ in guinea pigs or (ii) inhibitory effect on increasing of blood pressure induced by $STA_2$ in guinea pigs (intravenously and orally).

The results of the experiments carried out are shown in the following table I.

The methods for the experiments hereinbefore described are detailed as follows.

Inhibitory effect on increasing of blood pressure induced by $STA_2$ in guinea pigs: A 7% sodium bicarbonate buffer solution (pH 7.6 ~ 8.6) of each tested compounds was administered intravenously via carotid vein or orally to a urethane-anaesthetized male guinea pig weighing 250 ~ 350 g, at a rate of 10 - 1000 μg/kg animal body weight. And then guinea pigs were administered intravenously a solution of $STA_2$ in phosphate buffer (pH 7.4). Change of the blood pressure was measured at carotid artery and then inhibition ratio (%) was calculated.

Of the compounds of the general formula (I) of the present invention, some compounds have an antagonistic effect on thromboxane $A_2$, especially inhibitory effect on increasing of blood pressure, lasting for a long time, and have a weak agonistic effect on thromboxane $A_2$ (stimulatory effect on increasing of blood pressure).

For example, in standard laboratory test, the effects were possible to be confirmed by (i) inhibitory effect on increasing of blood pressure induced by $STA_2$ in guinea pigs (antagonistic effect) and (ii) stimulatory effect on increasing of blood pressure in guinea pigs (agonistic effect).

The results about a part of experiments carried out are shown in the following Table I and Figure 1 and 2.

Table I : (i) Inhibitory effects on increasing of blood pressure induced by $STA_2$ in guinea pigs and (ii) Stimulatory effect on increasing of blood pressure in guinea pigs

| | Example No. of compounds | Structure | (i) Duration of more than 50% inhibit on increasing of blood pressure induced by $STA_2$ in guinea pig (min) 100 μg/kg, p.o. | (ii) Effect of increasing of blood pressure in guinea pig (mmHg) 100 μg/kg, i.v. (n=2~4) |
|---|---|---|---|---|
| The compounds of the present invention | 1 | 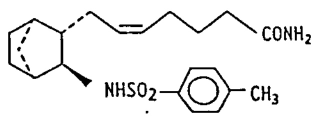 | >340 | 9 |
| | 2 | 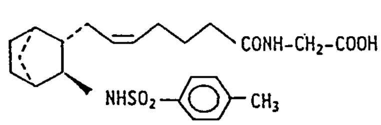 | >270 | 9 |
| | 2 (b) | 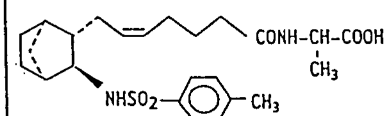 | >290 | 11 |
| | 2 (c) | 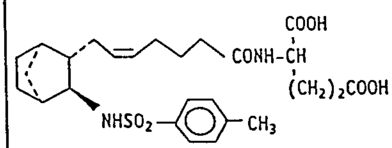 | >290 | 7 |
| | 3 | 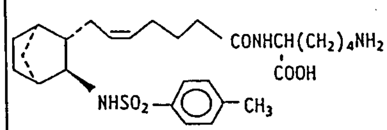 | 305 | 7 |

The methods for the experiments described in Table I and Figure 1 and 2 are detailed as follows.

Inhibitory effect on increasing of blood pressure induced by $TXA_2$ in guinea pigs and stimulatory effect on increasing of blood pressure in guinea pigs: A 7% sodium bicarbonate buffer solution (pH 7.6 ~ 8.6) of each tested compounds was administered intravenously via carotid vein or orally to a urethane-anaesthetized male guinea pig weighing 250 ~ 350 g, at a rate of 100 μg/kg animal body weight. Increasing of blood pressure was measured in guinea pigs administered test compounds intravenously. On the other hand, to guinea pigs administered test compounds orally, then a solution of $STA_2$ in phosphate buffer (pH 7.4) was administered. Change of the blood pressure was measured at carotid artery and then inhibition ratio (%) was calculated.

The foregoing results show that the compounds of the present invention possess an antagonistic effect on $TXA_2$. Some of them have an antagonistic effect on $TXA_2$, especially inhibitory effect on increasing of blood pressure, lasting for longer time than the compared compounds, and have a weak agonistic effect on $TXA_2$, i.e. stimulatory effect on increasing of blood pressure.

[Application for pharmaceuticals]

The compounds of the present invention, of the general formula (I), cyclodextrin clathrates thereof and non-toxic salts thereof have an antagonistic effect on $TXA_2$ such as an inhibitory effect on platelet aggregation, on contraction of smooth muscle or on increasing of blood pressure, and are, therefore, useful for prevention and/or treatment of inflammation, hypertension, thrombus, cerebral apoplexy, asthma, cardiac infarction, angina pectoris, cerebral infarction and death by acute cardiac disorders in mammals, in

particular in human, which are considred to be inudced by thromboxane $A_2$.

For the purpose hereinbefore described, the compounds of the present invention, of the general formula (I), cyclodextrin clathrates thereof and non-toxic salts thereof may normally be administered systemically or partially; usually by oral or parenteral administration. The doses to be administered is determined depending upon age, body weight, symptom, the desired therapeutic effect, the route of administration, and the duration of the treatment etc. In the human adult, the doses per person per dose are generally between 1 mg and 5 g, by oral administration up to several times per day, and between 10 $\mu$g and 1 g, by parenteral administration up to several times per day. As mentioned above, the doses to be used depend upon various conditions. therefore, there are cases in which doses lower than or greater than the ranges specified above may be used.

Solid compositions according to the present invention for oral administration include compressed tablets, dispersible powders and granules. In such solid compositions, one or more of the active compound-(s) is or are, admixed with at least one inert diluent such as lactose, mannitol, glucose, hydroxypropylcellulose, microcrystalline cellulose, startch, polyvinylpyrrolidone or magnesium metasilicate aluminate. The compositions may also comprise, as is normal practice, additional sustances other than inert diluents e.g. lubricating agents such as magnesium stearate, disintegrating agents such as cellulose calcium glycolate, stabilizing agent e.g. lactose and assistant for dissolving e.g. arginine, glutamic acid or aspartic acid. The tablets or pills may, if desired, be made into gastric film-coated or enteric film-coated tablets or pills, such as sugar-coated, gelatin-coated, hydroxypropyl cellulose-coated or hydroxypropylmethyl cellulose phthalate-coated tablets or pills; two or more of layers may be used. The compositions for oral administration also include capsules of absorbable material such as gelatin.

Liquid compositions for oral administration include pharmaceutically-acceptable emulsions, solutions, suspensions, syrups and elixirs containing inert diluents commonly used in the art such as distilled water or ethanol. Besides inert diluents such compositions may also comprise adjuvants such as wetting and suspending agents, and sweetening, flavouring, perfuming and presserving agents.

Other compositions for oral administration include spray compositions which may be prepared by known methods and which comprise one or more of the active compound(s).

Preparations for injection according to the present invention for parenteral administration include sterile aqueous or non-aqueous solutions, suspensions or emulsions. Example of aqueous solvents or suspending media are distilled water for injection and physiological salt solution. Examples of non-aqueous solvents or suspending media are propylene glycol, polyethylene glycol, vegetable oils such as olive oil, alcohols such as ethanol, POLYSORBATE 80 (registered Trade Mark). These compositions may also include adjuvants such as preserving, wetting, emulsifying, despersing agents, stabilizing agents (e.g. lactose) and assistant agent for dissolving (e.g. arginine, glutamic acid or aspartic acid). They may also be manufactured in the form of sterile solid compositions which can be dissolved in sterile water or some other sterile injectable medium immediately before use.

Other compositions for parenteral administration include liquids for external use, and endermic liniments such as ointments, suppositories for rectal administration and pessaries for vaginal administration whcih comprise one or more of the active compound(s) and may be prepared by known methods.

The following reference examples and examples illustrate the present invention, but not limit the present invention.

In the reference examples and examples, "bp", "mp", "TLC", "NMR", "IR" and "MS" represent "boiling point", "melting point", "Thin layer chromatography", "Nuclear magnetic reasonance spectrum", "Infrared absorption spectrum" and "Mass spectrum", respectively.

The solvents in the parentheses show the developing or eluting solvents and the rations of the solvents used are by volume in chromatographic separations.

Unless otherwise specified, "NMR" was measured in a chloroform-d ($CDC1_3$) solution and "IR" was measured by the liquid film method respectively.

In the structural formulae, 「cbz」 means 「benzyloxycarbonyl group」, 「Menth」 means 「menthyl group」, 「tosyl group」 means 「(4-methylphenyl)sulfonyl group」, 「mesyl group」 means 「methanesulfonyl group」, 「BMS」 means 「tert-butyldimethylsilyl group」 and 「THP」 means 「tetrahydropyran-2-yl group」.

And (±) in reference examples and examples represent the mixture of enantimers having different angles of rotation as generally used in the nomenclature. For example,

(±) 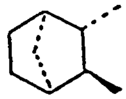

represents the mixture of

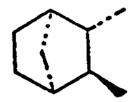

and

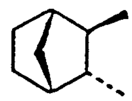

,

and * are added to indications of absolute configuration at the same time.

Reference example 1

(1R, 2S, 3S, 4S)-bicyclo [2.2.1] hept-5-ene-2,3-dicarboxylic acid di-(-)-menthyl ester

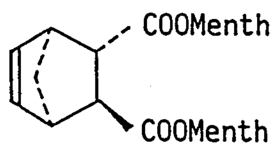

Di-(-)-menthyl fumarate (3.82g) dissolved in toluene (100 ml) was cooled to -78°C. Diethylaluminum chloride (1.49g) was added dropwise to the mixture. The mixture was stirred for 30 minutes. 1,3-cyclopentadiene (660mg) distilled freshly was added to the obtained reaction solution. The mixture was stirred for 30 minutes at a temperature of -78°C and then the temperature of the solution was raised to -20°C by degrees. 2N hydrochloric acid (200 ml) was added to the reaction mixture. The organic layer was separated. The water layer was extracted twice with ether. The extract was washed with water, dried over sodium sulfate and then evaporated to obtain the title compound (4.2g) having the following physical data:
NMR($CCl_4$ solution): δ 6.20 (2H, S), 4.60 (2H, m), 3.40 (2H, m), 2.55 (2H, m).

Reference example 2

The mixture of
[(1R, 2S, 3S, 4S)-3-carboxybicyclo [2.2.1] hept-5-en-2-yl] carboxylic acid-(-)menthyl ester and the corresponding (1S, 2S, 3S, 4R) compound

The mixture of

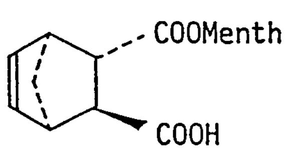

and

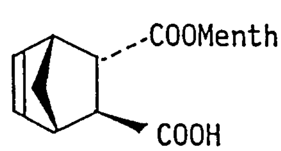

2N aqueous solution of sodium hydroxide (1mℓ) was added to dimenthyl ester (1.0g, prepared in reference example 1 ) dissolved in ethanol (20mℓ). The mixture was refluxed for 30 minutes. The reaction mixture was evaporated. Water (50mℓ) was added to the residue. The mixture was extracted with ethyl acetate. The water layer was acidified by adding 1N hydrochloric acid. The mixture was extracted with ethyl acetate. The extract was washed with water, dried over magnesium sulfate and evaporated. The residue was purified by column chromatography on silica gel (ethyl acetate: n-hexane = 2:3) to obtain the title compound (320mg) having the following physical data:

NMR ($CCl_4$ solution): δ 6.17 (2H), 4.36 (2H), 3.5 ~ 2.5 (4H, m).

Reference example 3

The mixture of [(1R, 2S, 3S, 4S)-3-benzyloxycarbonylamino bicyclo [2.2.1] hept-5-en-2-yl] carboxylic acid menthyl ester and the corresponding (1S, 2S, 3S, 4R) compound

The mixture of

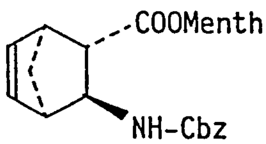

and

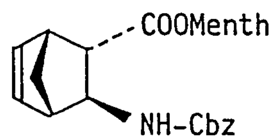

Triethylamine (120mg) was added to the half ester (320mg, prepared in reference example 2 ) dissolved in acetone (5mℓ). The solution was cooled to 0 ° C. Ethyl chloroformate (130mg) was added dropwise to the solution. The mixture was stirred for 20 minutes. Sodium azide (130mg) dissolved in water (1mℓ) was added to the mixture cooled to 0 ° C. The mixture was stirred with keeping a same temperature for 30 minutes. The water was added to the reaction mixture. The mixture was extracted three times with toluene to obtain a toluene solution of the corresponding 3-azidecarbonyl body. The solution was dried over magnesium sulfate and then refluxed for 2 hours to obtain the corresponding 3-isocyanate. Benzylalcohol (108mg) and a catalyst amount of tributylamine were added to the obtained reaction mixture. The mixture was refluxed for 3 hours. The reaction mixture was evaporated. The residue was purified by column chromatography on silica gel (ethyl acetate: n-hexane = 1:6) to obtain the title compound (mixture, 340mg) having the following physical data.

NMR: δ 7.2 (5H), 6.3 (2H), 4.95 (2H, S), 4.35 (2H, m), 2.95 (2H, m).

Reference example 4

[(1R, 2S, 3S, 4S)-3-benzyloxycarbonylamino bicyclo [2.2.1] hept-5-en-2-yl] carboxylic acid menthyl ester or the corresponding (1S, 2S, 3S, 4R) compound

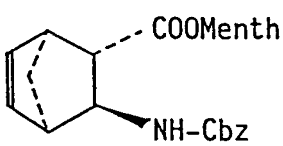

(a)

or

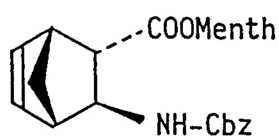

(b)

Two isomers obtained in reference example 3 were purified by column chromatography on silica gel (ethyl acetate: n-hexane = 3:7) to obtain the title two compounds having the following physical data.

(a) (1R, 2S, 3S, 4S) isomer:
TLC (methylene chloride: n-hexane: ether = 12:4:1) : Rf = 0.35.
(b) (1S, 2S, 3S, 4R) isomer:
TLC (methylene chloride: n-hexane: ether = 12:4:1) : Rf = 0.32.

Reference example 5

[(1R, 2S, 3S, 4S)-3-tosylaminobicyclo [2.2.1] heptan-2-yl]acetoaldehyde

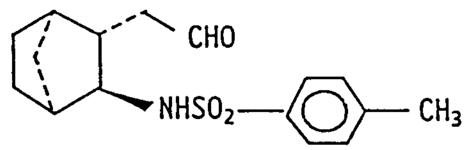

12N hydrochloric acid (0.41mℓ) and palladium-carbon (content: 10%, 200mg) were added to 2-menthyloxycarbonyl-3-benzyloxycarbonylamino compound (prepared in reference example 4 (a), 1.08g) dissolved in anhydrous ethanol (10mℓ). The mixture was stirred in an atmosphere of hydrogen, at a room temperature, for 2 hours. The catalyst was removed from the mixture by filtration. The filtrate was evaporated to obtain hydrochloric acid salt of the corresponding amine. The obtained amine was dissolved in pyridine (10mℓ). Tosyl chloride (1.24g) and triethylamine (0.45mℓ) were added to the mixture with cooling in an ice-bath. The mixture was stirred at a room temperature for 12 hours. The yellow-orange reaction mixture was poured into 4N hydrochloric acid (50mℓ). The mixture was extracted with ethyl acetate (100mℓ). The extract was washed with a saturated aqueous solution of sodium bicarbonate, followed by a brine, dried over magnesium sulfate and evaporated. The residue was purified by column chromatography on silica gel (ethyl acetate: n-hexane = 1:2) to obtain [(1R, 2S, 3S, 4S)-3-tosylaminobicyclo [2.2.1] heptan-2-yl]carboxylic acid ethyl ester.

Next, in an atmosphere of argon, the ethyl ester obtained before (860mg) dissolved in anhydrous THF (5mℓ) was added dropwise to lithium aluminum hydride (LAH, 186mg) suspended in anhydrous tetrahydrofuran (THF, 15mℓ) at a room temperature. The mixture was stirred for 30 mins. The reaction mixture was cooled to 0 ° C. The mixture of THF-water (5:2) was added to the mixture to quench excess LAH. The occurred mixture such as rice gruel was dissolved by adding 1N hydrochloric acid (30mℓ). The mixture was extracted three times with ethyl acetate (20mℓ). The extract was dried over magnesium sulfate and then evaporated to obtain the corresponding alcohol (white solid, 683mg).

Next, alcohol described above (309mg) was dissolved in dimethyl sulfoxide (DMSO, 5mℓ). Triethylamine (0.5mℓ) was added to the solution. Trioxide-pyridine complex (477mg) dissolved in DMSO (2mℓ) was added dropwise to the solution. The mixture was stirred at a room temperature for 1 hour, poured into ice-water (30mℓ) and extracted twice with ethyl acetate (20mℓ). The extract was dried over magnesium sulfate and then evaporated.

The obtained residue was purified by column chromatography on silica gel (ethyl acetate: methylene chloride = 1:10) to obtain [(1R, 2S, 3S, 4S)-3-tosyl aminobicyclo [2.2.1] heptan-2-yl] aldehyde (287mg, white solid).

Next, 1.4 M solution (2.1mℓ) of n-butyllithium in hexane was added dropwise to diisopropylamine (0.5mℓ) dissolved in tetrahydrofuran (THF, 3mℓ) at a temperature of 0 ° C. The mixture was stirred for 15 minutes to obtained a solution of lithiumdiisopropylamide (LDA).

Next, a solution of LDA prepared before was added dropwise to 4-chlorophenoxymethyl-triphenylphosphoniumchloride (1.32g) suspended in THF (10mℓ) at a temperature of 0 ° C to obtain a deep red solution.

Aldehyde (287mg) prepared before dissolved in THF (5mℓ) was added dropwise to the solution. The solution was stirred at a temperature of 0 ° C for 30 minutes. The reaction mixture was poured into ice-water (30mℓ). The mixture was extracted twice with ethyl acetate (50mℓ). The extract was dried over magnesium sulfate and evaporated. The residue was purified by column chromatography on silica gel (ethyl acetate: n-hexane = 1:3) to remove polar substances. Enol ether obtained before was concentrated 1.4- dioxane (10ml) and 4N hydrochloric acid (2ml) were added to the residue The mixture was stirred at 80 ° C for 40 minutes. Ice cooled 10% aqueous solution of sodium hydroxide (30ml) was added to the mixture. The mixture was extracted twice with ethyl acetate (50ml). The extract was dried over magnesium sulfate and evaporated to obtaine the title compound having the following physical data. The compound was used in next step without purification.

NMR: δ 9.75 (1H, bs), 7.75 (2H, d), 7.30 (2H, d);
MS: m/e 307 ($M^+$), 289, 278.

Reference example 6

(5Z)-7-[(1R, 2S, 3S, 4S)-3-tosylaminobicyclo [2.2.1] heptan-2-yl]hept-5-enoic acid

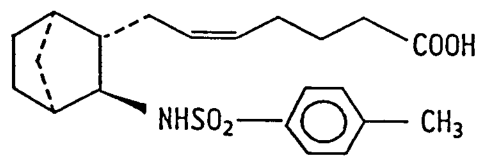

Potassium tert-butoxide (740mg) was added to 4-carboxybutyltriphenylphosphoniumbromide (1.33g) suspended in toluene (10mℓ). The mixture was stirred at a temperature of 80 °C for 40 minutes. The obtained reaction solution was cooled to 0 °C. [(1R, 2S, 3S, 4S)-3-tosylaminobicyclo [2.2.1] heptan-2-yl]-acetoaldehyde (270mg; prepared in reference example 5 ) dissolved in toluene (3mℓ) was added dropwise to the solution. The solution was stirred at a temperature of 0 °C for 15 minutes. The reaction mixture was poured into ice-water. The mixture was extracted three times with ether (20mℓ) to remove neutral substances and basic substances. The water layer was acidified with 1N hydrochloric acid (10mℓ). The solution was extracted twice with ethyl acetate (30mℓ). The extract was dried over magnesium sulfate and evaporated. The residue was purified by column chromatography on silica gel (methylene chloride: methanol = 100:1→25:1) to obtain the title compound (222mg) having the following physical data:

TLC (methylene chloride: methanol = 10:1): Rf 0.45;

NMR: δ 7.75 (2H, d), 7.28 (2H, d), 5.26 (2H, m), 5.11 (1H, d), 3.00 (1H, broad), 2.42 (3H, S), 2.37 (2H, t), 2.16~0.87 (15H);

MS: m/e 391 ($M^+$), 373;

m.p.: 77~78 °C;

feature: white crystal.

Example 1

(5Z)-7-[(1R, 2S, 3S, 4S)-3-tosylaminobicyclo [2.2.1] heptan-2-yl]hept-5-enamide

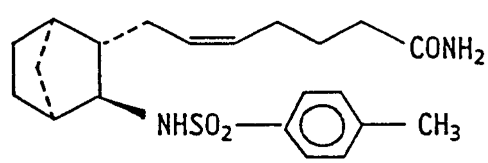

Oxalyl chloride (2mℓ) was added to (5Z)-7-[(1R, 2S, 3S, 4S)-3-tosylaminobicyclo [2.2.1] heptan-2-yl]-hep-5-tenoic acid (100mg, prepared in reference example 6). The mixture was stirred at a room temperature for 1 hour. Excess oxalyl chloride was removed from the mixture in vacuo to obtain an acid chloride. This acid chloride was dissolved in methylene chloride (5mℓ). The solution was cooled to 0 °C and became turbid by blowing ammonia gas through the solution. The mixture was stirred at a room temperature for 30 mins. The insoluble substance was removed by filtration. The filtrate was concentrated. The residue was purified by column chromatography on silica gel (ethyl acetate: hexane = 1:1→5:1) to obtain the title compound 81.4 mg having the following physical data:

TLC (ethyl acetate: hexane = 5:1) : Rf 0.35;

NMR: δ 7.76 (2H, d), 7.27 (2H, d), 5.80 (1H, broad), 5.62 (1H, broad), 5.61 (1H, d), 5.33 (2H, m), 3.04 (1H, broad), 2.43 (3H, S);

MS: m/e 390 ($M^+$), 332, 290, 278, 235, 218;

feature: colorless wax.

Example 1(a) and 1(b)

The compounds shown in Table II was obtained by the same procedure as example 1, with using suitable amines instead of ammonia gas in example 1(a) and 1(b).

Table 11

| Example No. | Structural formula | Name | TLC | MS(m/e) |
|---|---|---|---|---|
| 1(a) | 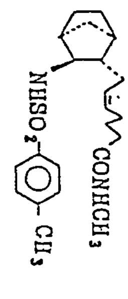 | (5Z)-N-methyl-7-[(1R,2S,3S,4S)-3-tosylaminobicyclo[2.2.1]heptan-2-yl]hept-5-enamide | Rf 0.35 (ethyl acetate: n-hexane =5:1) | 404(M+),332,318,290,278,264,250 |
| 1(b) | 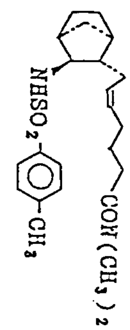 | (5Z)-N,N-dimethyl-7-[(1R,2S,3S,4S)-3-tosylaminobicyclo[2.2.1]heptan-2-yl]hept-5-enamide | Rf 0.35 (ethyl acetate: n-hexane =5:1) | 418(M+),374,332,318,290,264,218 |

Example 2

Amide of (5Z)-7-[(1R, 2S, 3S, 4S)-3-tosylaminobicyclo [2.2.1] heptan-2-yl]hept-5-enoic acid and glycine

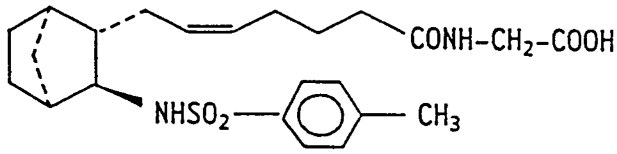

(5Z)-7-[(1R, 2S, 3S, 4S)-3-tosylaminobicyclo [2.2.1] heptan-2-yl]hept-5-enoic acid (100mg, prepared in reference example 6.), glycine tert-butyl ester hydrochloride (65.3mg), 2-chloro-1-methylpyridinium iodide (100mg) and triethylamine (0.22mℓ) were dissolved in methylene chloride (5mℓ). The mixture was stirred at a room temperature for 4 hours. The solvent was removed. The residue was purified by column chromatography on silica gel (ethyl acetate: hexane = 1:2) to obtain the condensed compound. Methylene chloride (2mℓ) and trifluoroacetic acid (1mℓ) were added to the compound. The solution was stirred overnight and then concentrated. Trifluoroacetic acid was removed by boiling with toluene. The residue was purified by column chromatography on silica gel (methanol: methylene chloride = 1:10) to obtain the title compound (56mg) having the following physical data: TLC (methanol: methylene chloride = 1:10): Rf 0.10;
NMR: δ 7.72 (2H, d), 7.31 (2H, d), 6.30 (1H, t), 6.08 (1H, d), 5.30 (2H, m), 4.20 (1H, dd), 3.95 (1H, dd), 2.84 (1H, d), 2.43 (3H, s);
MS: m/e 448 ($M^+$), 332, 307, 293, 277, 265;
feature: colorless amorphous.

Example 7(a) ~ 7(c)

The compounds shown in Table III were obtained by the same precedure as described in example 2, with using the corresponding acid and suitable amino acid ester(pyrrolidine in example 7(a)).

Table III

| Example No. | Structural formula | Name | TLC | MS(m/e) |
|---|---|---|---|---|
| 2(a) | 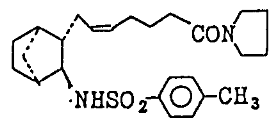 | amide of (5Z)-7-[(1R,2S,3S,4S)-3-tosylaminobicyclo[2.2.1]heptan-2-yl]hept-5-enoic acid and pyrrolidine | Rf 0.35 ethyl acetate: n-hexane =3:1) | 444(M+),332,289, 272,261,236,218 |
| 2(b) | 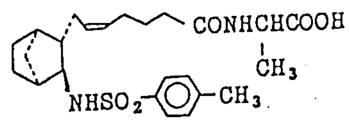 | amide of (5Z)-7-[(1R,2S,3S,4S)-3-tosylaminobicyclo[2.2.1]heptan-2-yl]hept-5-enoic acid and alanine | Rf 0.15 (methylene chloride: methanol =10:1) | 462(M+),444,375, 332,307,291,279 |
| 2(c) | 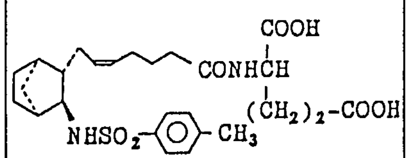 | amide of (5Z)-7-[(1R,2S,3S,4S)-3-tosylaminobicyclo[2.2.1]heptan-2-yl]hept-5-enoic acid and glutamic acid | Rf 0.10 (acetic acid: methanol: methylene chloride 1:10:100) | 502(M-$H_2O$),391, 373,347,332,319 |

Example 3

Amide of (5Z)-7-[(1R, 2S, 3S, 4S)-3-tosylaminobicyclo [2.2.1] heptan-2-yl]hept-5-enoic acid and lysine

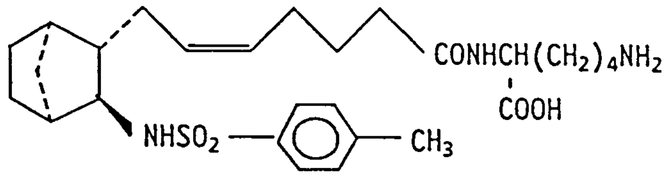

Oxalyl chloride (2mℓ) was added to (5Z)-7-[(1R, 2S, 3S, 4S)-3-tosylaminobicyclo [2.2.1] heptan-2-yl]-hept-5-enoic acid (100mg, prepared in reference example 6 ). The mixture was stirred at a room temperature for 1 hour. Excess oxalyl chloride was removed in vacuo to obtain an acid chloride.

Next, tetrahydrofuran (4mℓ) and 4N aqueous solution of sodium hydroxide (1mℓ) were added to ε-t-butylcarbonyl-L-lysine (492mℓ). The solution was cooled to 0°C. Acid chloride prepared before dissolved in anhydrous tetrahydrofuran (2mℓ) was added dropwise to the solution. The reaction solution was stirred enough for 20 mins, acidified with 1N hydrochloric acid and then extracted with ethyl acetate. The ethyl acetate layer was dried over magnesium sulfate, filtrated and concentrated. The residue was purified by column chromatography on silica gel (methanol: methylene chloride = 1:20→1:10) to obtain the condensed compound. The obtained condensed compound was dissolved in methylene chloride (2mℓ). Trifluoroacetic acid (1mℓ) was added to the solution. The mixture was stirred at a room temperature for 3 hours. The solvent was removed by evaporation. The residue was purified by column chromatography on silica gel (methanol: methylene chloride = 1:10→1:5→2:3) to obtain the title compound (97.4mg) having the following physical data.

TLC (methanol: methylene chloride = 2:3): Rf = 0.20;

NMR: δ 7.73 (2H, d), 7.31 (2H, d), 5.23 (2H, m), 2.91 (4H, m), 2.43 (3H, s);

MS: m/e 501 (M-18), 346, 218;

feature: pale yellow amorrhous.

**Claims**

**1.** A sulfonamide derivative of the general formula:

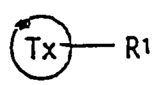 (I)

wherein, $R^1$ represents

(i) $COOR^{11}$,

(ii) $CH_2OR^{12}$ or

(iii) $CONR^{13}R^{14}$,

wherein

$R^{11}$ represents a carbocyclic ring unsubstituted or substituted by an alkyl or alkoxy group of from 1 to 4 carbon atom(s) or halogen atom, or steroid,

$R^{12}$ represents a hydrogen atom or $COR^{15}$,

$R^{13}$ and $R^{14}$ each represent a hydrogen atom or alkyl group of form 1 to 4 carbon atom(s) or $NR^{13}R^{14}$ represents an amino acid residue or heterocyclic ring, or

$R^{15}$ represents an alkyl group of from 1 to 4 carbon atom(s) or phenyl group,

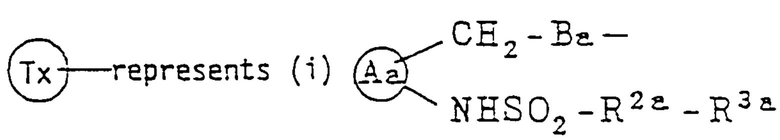 (A)

wherein

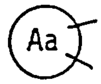

represents

(i) 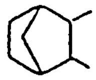 (Aa-1),

(ii) 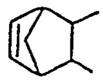 (Aa-2),

(iii) 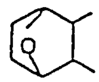 (Aa-3),

(iV) 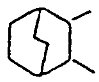 (Aa-4),

(V) 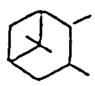 (Aa-5),

(Vi) 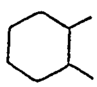 (Aa-6),

(Vii) 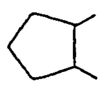 (Aa-7),

(Viii) 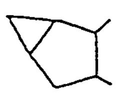 (Aa-8),

(iX) 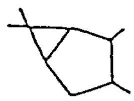 (Aa-9),

(X) 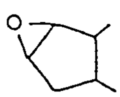 (Aa-10),

(Xi) 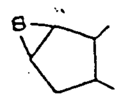 (Aa-11),

Ba represents

(i) $-CH_2-CH_2-(CH_2)m-$ (Ba-1),
(ii) cis-$CH=CH-(CH_2)m-$ (Ba-2),
(iii) $-CH_2-O-(CH_2)m-$ (Ba-3),
(iV) $-S-(CH_2)m-$ (Ba-4) or
(V)

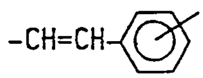 (Ba-5)

wherein m represents an integer of from 1 to 6, and the configuration of a double bond is E, Z or EZ and a phenylene group represetns an o-, m- or p-phenylene group in the formula (Ba-5),

$R^{2a}$ represents a bond or an alkylene group of from 1 to 4 carbon atom(s),
$R^{3a}$ represents a carbocyclic or heterocyclic ring unsubstituted or substituted by from one to three of an alkyl group of from 1 to 4 carbon atom(s), alkoxy group of from 1 to 4 carbon atom(s), hydroxy group, carboxyl group, ciano group, halogen atom or nitro group, or
$R^{2a}$ represents an alkyl group of from 1 to 12 carbon atom(s) together with $R^{3a}$,

cyclodextrin clathrates thereof, or non-toxic salts thereof in case that $NR^{13}R^{14}$ represents an amino acid residue.

**2.** A derivative according to claim 1, wherein

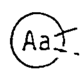

represents the formula (Aa-1)

**3.** A derivative according to claim 2, which is
(5Z)-7-(3-tosylaminobicyclo[2.2.1]heptan-2-yl)hept-5-enamide,
(5Z)-N-methyl-7-(3-tosylaminobicyclo[2.2.1]heptan-2-yl)hept-5-enamide,
(5Z)-N,N-dimethyl-7-(3-tosylaminobicyclo[2.2.1]heptan-2-yl)hept-5-enamide,
amide of (5Z)-7-(3-tosylaminobicyclo[2.2.1]heptan-2-yl)hept-5-enoic acid and pyrrolidine,
amide of (5Z)-7-(3-tosylaminobicyclo[2.2.1]heptan-2-yl)hept-5-enoic acid and glycine,
amide of (5Z)-7-(3-tosylaminobicyclo[2.2.1]heptan-2-yl)hept-5-enoic acid and alanine,
amide of (5Z)-7-(3-tosylaminobicyclo[2.2.1]heptan-2-yl)hept-5-enoic acid and glutamic acid or
amide of (5Z)-7-(3-tosylaminobicyclo[2.2.1]heptan-2-yl)hept-5-enoic acid and lysine.

**4.** A process for the preparation of the compounds of the general formula:

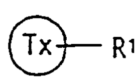 (I)

(wherein,

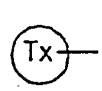

and $R^1$ represent the same meaning as hereinbefore defined.)

which is characterized by

subjecting the compound of the general formula:

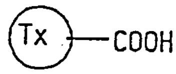

(Ib)

(wherein

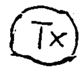

has the meaning as hereinbefore defined) to the reaction to form an amide,
subjecting the compound of the general formula:

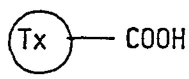

(Ib)

(wherein, a symbol represents the same meaning as hereinbefore described)
to esterification,
subjecting the compound of the the general formula:

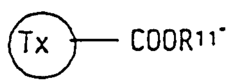

(Id)

(wherein, $R^{11-}$, represents an alkyl group of from 1 to 8 carbon atom(s) and the other symbols represent the same meaning as hereinbefore defined)
to reduction,
subjecting the compound of the general formula:

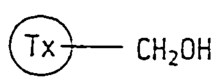

(Ie)

(wherein, the symbol represents the same meaning as hereinbefore defined)
to acylation

**5.** A pharmaceutical composition for the prevention and/or treatment of hypertension, thrombus, cerebral apoplexy, asthma, cardiac infarction, angina pectoris, cerebral infarction, and acute cardiac deseases, which comprises, as active ingredient, an effective amount of at least one compound of the general formula ( I ) depicted in claim 1 wherein various symbols are as defined in claim 1, or a cyclodextrin clathrate thereof, or, when
$NR^{13}R^{14}$ represents an amino acid residue, a non-toxic salt thereof, together with a pharmaceutical carrier or coating.

**6.** A compound as claimed in claim 1, a cyclodextrin clathrate thereof, or when $NR^{13}R^{14}$ represents a non-toxic salt thereof, for use in manufacture of a medicament for the prevention and/or treatment of hypertension, thrombus, cerebral apoplexy, asthma, cardiac infarction, angina pectoris, cerebral infarction, and acute cardiac deseases.

Fig. 1 Inhibitory ratio on increasing of blood pressure induced by $STA_2$ in guinea pig

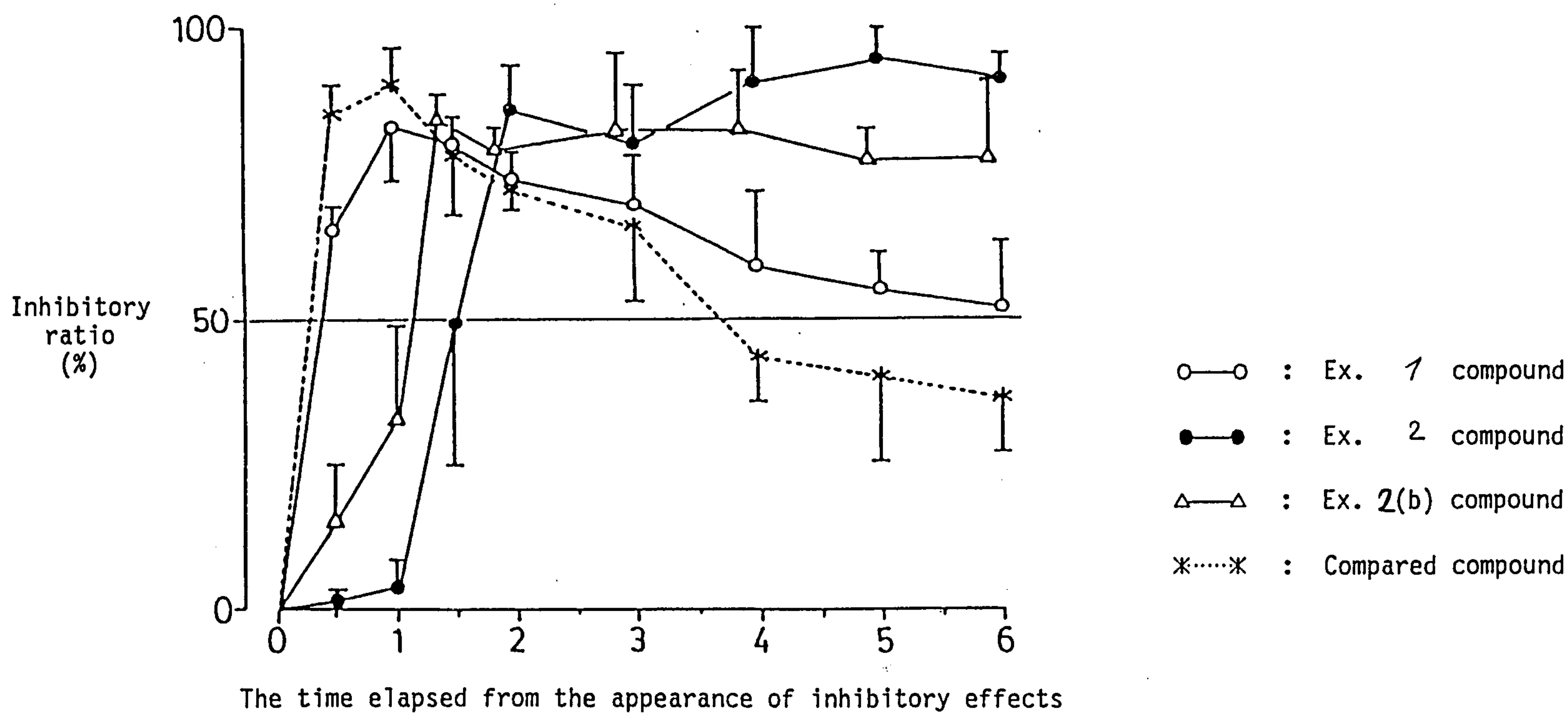

Fig. 2 Inhibitory ratio on increasing of blood pressure induced by $STA_2$ in guinea pig

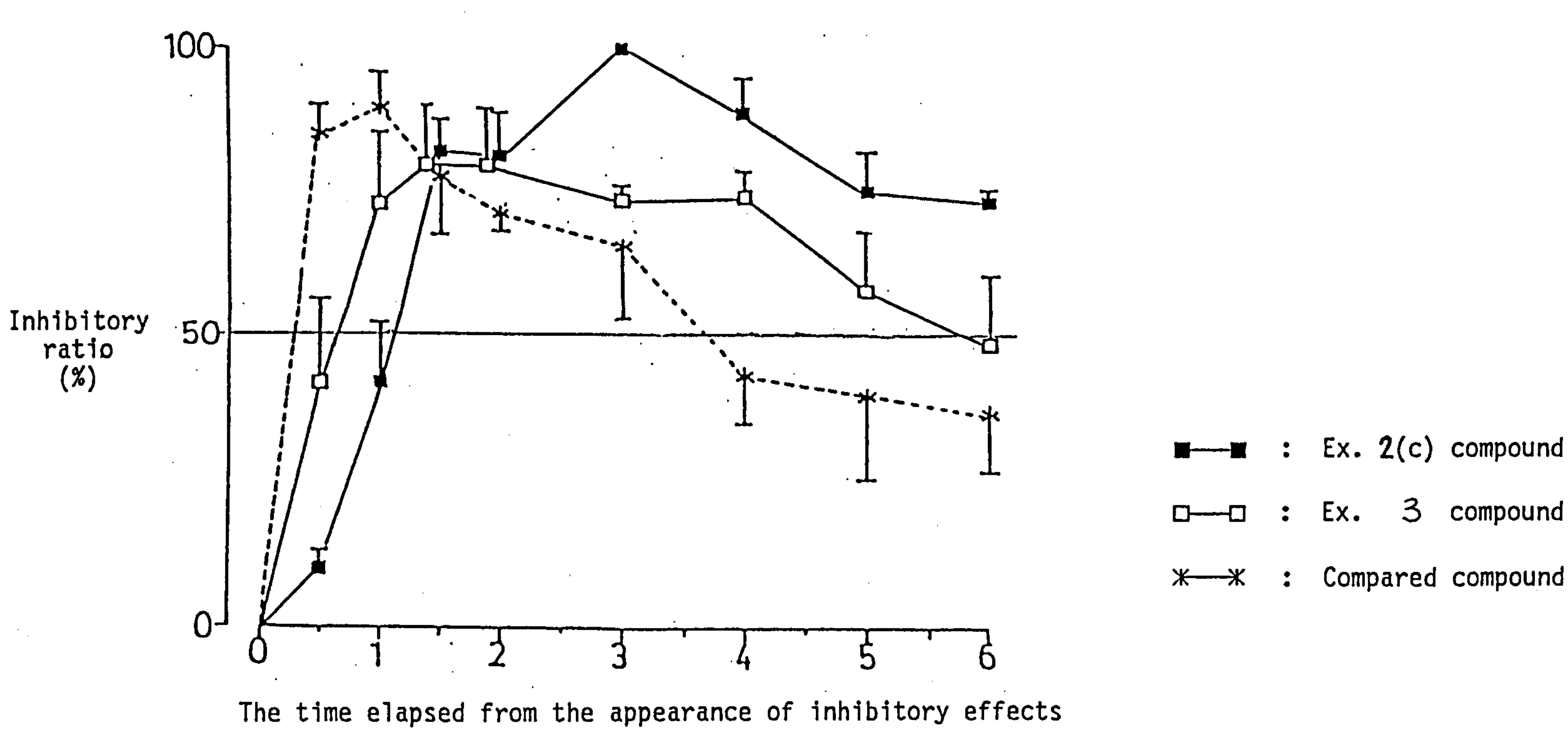

European Patent Office

# EUROPEAN SEARCH REPORT

Application Number

EP 92 11 2113

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4 ) |
|---|---|---|---|
| D,Y | EP-A-0 226 346 (SHIONOGI & CO., LTD.)<br>* the whole document *<br>--- | 1-6 | C07C405/00<br>C07C311/20<br>C07C311/10 |
| Y | GB-A-2 039 480 (R.L. JONES AND N.H. WILSON)<br>* the whole document, particularly page 1, lines 70-85 *<br>----- | 1-6 | C07C311/12<br>C07D303/02<br>C07D493/08<br>C07D331/02<br>A61K31/557<br>//(C07D493/08, 307:00,307:00) |
| | | | TECHNICAL FIELDS SEARCHED (Int. Cl.4 ) |
| | | | C07C<br>C07D |

The present search report has been drawn up for all claims

| Place of search | Date of completion of the search | Examiner |
|---|---|---|
| THE HAGUE | 21 AUGUST 1992 | ALLARD M.S. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document